# DEMANDE DE BREVET EUROPEEN

(11) **EP 3 587 583 A1**
(43) Date de publication de la demande: **01.01.2020**
(21) Numéro de dépôt: 19178488.3
(22) Date de dépôt: 05.06.2019
(51) Int. Cl.: C12P 19/02, C12P 19/14, C12P 7/10, B01D 29/09, C12M 1/00, C12M 1/33, C13B 20/16

(54) **PROCEDE DE TRAITEMENT DE BIOMASSE LIGNO-CELLULOSIQUE**

(30) Priorité: 27.06.2018 FR 1855789
(71) Demandeur: IFP Energies nouvelles, 92852 Rueil-Malmaison Cedex (FR); Institut National de la Recherche Agronomique, 75338 Paris Cedex 07 (FR); Agro Industries Recherche Et Developpement, 51110 Pomacle (FR)
(72) Inventeur: AYMARD, Caroline, 92852 RUEIL-MALMAISON CEDEX (FR); ROUSSET, Romain, 92852 RUEIL-MALMAISON CEDEX (FR); PEROTTA, Larissa, 92508 RUEIL MALMAISON CEDEX (FR); KNOSPE, Emilia, 92508 RUEIL MALMAISON CEDEX (FR)
(74) Mandataire: IFP Energies nouvelles

(57) **Abrégé**

L'invention concerne un procédé de traitement d'une biomasse lignocellulosique, avec :
a. Prétraitement de la biomasse par au moins une opération de cuisson ou d'explosion à la vapeur, afin d'obtenir un substrat prétraité,
b. Séparation liquide/solide sur au moins une partie du substrat prétraité, comprenant deux sous-étapes successives :
- une sous-étape amont de contactage b1 du mélange solide/liquide mise en oeuvre par un mélangeur (M) en continu utilisant un fluide de mélange
- une sous-étape aval b2 d'extraction/lavage mise en oeuvre par un filtre en continu, utilisant un fluide de lavage, pour obtenir une phase solide enrichie en solide et une pluralité de phases liquides enrichies en liquide, avec un recyclage au moins partiel d'une phase liquide extraite du filtre en entrée du mélangeur en tant que fluide de mélange.

## Description

### Domaine de l'invention

L'invention concerne un procédé de traitement de biomasse lignocellulosique pour produire des jus sucrés dits de seconde génération (2GCes jus sucrés peuvent être utilisés pour produire d'autres produits par voie chimique ou voie biochimique/fermentaire (par exemple, des alcools comme l'éthanol, le butanol ou d'autres molécules, par exemple des solvants tels que l'acétone et d'autres molécules biosourcées...).

### Art antérieur

La biomasse lignocellulosique représente une des ressources renouvelables les plus abondantes sur terre. Les substrats considérés sont très variés, ils concernent à la fois des substrats ligneux comme différents bois (feuillus et résineux), des coproduits issus de l'agriculture (pailles de blé, pailles de riz, rafles de maïs, etc...) ou d'autres industries agroalimentaires, papetières, etc... La biomasse lignocellulosique est composée de trois principaux polymères : la cellulose (35 à 50%), qui est un polysaccharide essentiellement constitué d'hexoses ; l'hémicellulose (20 à 30%), qui est un polysaccharide essentiellement constitué de pentoses ; et la lignine (15 à 25%), qui est un polymère de structure complexe et de haut poids moléculaire, composé d'alcools aromatiques reliés par des liaisons éther. Ces différentes molécules sont responsables des propriétés intrinsèques de la paroi végétale et s'organisent en un enchevêtrement complexe. Parmi les trois polymères de base qui intègrent la biomasse lignocellulosique, la cellulose et l'hémicellulose sont ceux qui permettent la production de jus sucrés 2G.

Le procédé de transformation biochimique de la lignocellulosique en jus sucrés 2G comprend notamment une étape de prétraitement et une étape d'hydrolyse enzymatique par un cocktail enzymatique. Ces procédés comportent aussi le plus souvent une étape d'imprégnation avant le prétraitement. Les jus sucrés issus de l'hydrolyse peuvent être utilisés/valorisés tels quels ou éventuellement être traités ultérieurement, par exemple dans un procédé fermentaire ou chimique. Le plus souvent, le procédé global comprend des étapes de séparation intermédiaires et/ou une étape de purification du produit final.

Le prétraitement permet de modifier les propriétés physico-chimiques de la biomasse lignocellulosique afin de rendre accessible la cellulose aux enzymes et atteindre une bonne réactivité en hydrolyse enzymatique. De nombreuses technologies de prétraitement existent et permettent la mise en température de la biomasse dans des conditions chimiques variées. Le prétraitement peut être effectué avec ou sans ajout de produits acides ou basiques. Il peut aussi être effectué dans un solvant comme l'eau ou un produit organique comme, par exemple, de l'alcool (procédé organosolv) mais aussi dans un milieu peu diluant comme de la vapeur. Ce prétraitement peut aussi avoir une étape physique comme un défibrage ou une détente explosive dans le cadre d'une explosion à la vapeur. Ce prétraitement peut aussi avoir plusieurs étapes permettant d'optimiser le procédé global, comme par exemple, une cuisson acide suivie d'une explosion à la vapeur ou deux explosions à la vapeur consécutives. Les prétraitements ci-après regroupés sous le terme générique de « cuisson » concernent des traitements en conditions diluées de la biomasse, et regroupent les cuissons acides, les cuissons alcalines, les cuissons « organosolv». Ce dernier procédé concerne un prétraitement en présence d'un ou plusieurs solvants organiques et généralement d'eau. Le solvant peut être un alcool (éthanol), un acide type acide acétique, acide formique, ou encore l'acétone. Les procédés « organosolv pulping » conduisent à une solubilisation au moins partielle de la lignine, une solubilisation partielle des hémicelluloses. On a alors deux flux en sortie : le substrat prétraité avec cellulose, hémicellulose et lignine résiduels et la phase solvant qui contient la ligne solubilisée et une partie des hémicellulose. Il y a généralement une étape de régénération du solvant qui permet d'extraire un flux de lignine. Certains traitements « organosolv pulping » (notamment avec de l'éthanol) sont couplés avec l'ajout d'un acide fort (du type H₂SO₄). On peut envisager également d'avoir une mise en contact de la biomasse avec le solvant via un réacteur d'imprégnation avant la phase de cuisson ou d'avoir une mise en contact de la biomasse avec le catalyseur acide avant d'effectuer une cuisson « organosolv pulping ».

Le prétraitement de type explosion à la vapeur est différent d'un traitement de type cuisson en ce sens que la biomasse est concentrée, et soumise à une quantité proportionnellement faible de vapeur d'eau. Le plus souvent, l'explosion à la vapeur en condition acide est préférée, car elle permet un bon compromis entre l'hydrolyse acide de l'hémicellulose et la réactivité de la cellulose en hydrolyse enzymatique, avec une hydrolyse presque totale de l'hémicellulose et une amélioration importante de l'accessibilité et la réactivité de la cellulose aux enzymes. Ce prétraitement peut être précédé d'autre(s) traitement(s) (broyage, imprégnation, cuisson etc...).

Différentes configurations sont reportées par exemple dans le document «Production of bioéthanol from lignocellulosic materials via the biochemical pathway: A review », M. Balat, Energy Conversion and Management 52 (2011) 858-875, ou encore dans le document « Bioethanol production from agricultural wastes : an overview », N. Sarkar, S. Kumar Ghosh, S. Bannerjee, K. Aikat, Renwable Energy 37 (2012) 19-27.

On note donc que ce type de procédé, cuisson ou explosion à la vapeur, requiert une transformation de la biomasse brute en un substrat prétraité réactif (appelé aussi marc prétraité), avant de démarrer les étapes suivantes de conversion à proprement dites. Après ce prétraitement, des sucres (sucres en C5 et C6) se retrouvent dans un jus sucré imprégnant le marc prétraité. La récupération de ce jus pourrait être intéressante pour une valorisation dans le reste du procédé de transformation de la biomasse ou dans un autre procédé (en parallèle) ou pour une commercialisation tel quel du jus sucré. Il y a donc un intérêt à extraire ces jus du marc prétraité, avant que le marc prétraité en question, qui sera alors enrichi en matière solide, ne subisse les traitements suivants.

L'étape de prétraitement est souvent précédée d'une étape d'imprégnation. Elle est suivie d'une étape d'hydrolyse enzymatique utilisant un cocktail enzymatique. Dans certains cas, ces étapes précédentes sont suivies d'une étape de fermentation éthanolique des sucres libérés et d'une étape de purification des produits de fermentation. Sous certaines configurations du schéma de procédé, les étapes d'hydrolyse enzymatique et de fermentation peuvent avoir lieu dans un même réacteur, dans une configuration de fermentation appelée SSF (Simultaneous Saccharification and Fermentation). Quand ces deux étapes du procédé sont séparées, on parle alors d'un schéma du type SHF (Separated Hydrolysys and Fermentation). Des exemples sont donnés par le document "Ethanol from lignocellulosics: A review of the economy", M. von Silvers et G. Zacchi, Bioresource Technology 56 (1996) 131-140.

Dans ces étapes aussi, on peut avoir des produits sous forme de mélange solide/liquide, par exemple les produits dits hydrolysats obtenus après l'hydrolyse enzymatique, où il peut être intéressant d'opérer une séparation solide/liquide, d'extraire la phase liquide, par exemple pour utiliser les jus produits dans une étape ultérieure sensible à la présence de solide, par exemple en cas de sensibilité du micro-organisme fermentaire mis en oeuvre dans une étape ultérieure de fermentation, ou en cas de recyclage du micro-organisme autour de cette étape de fermentation.

On voit donc qu'à différentes étapes des procédés de traitement de biomasse, on a affaire à des produits sous forme de mélanges solide/liquide, et qu'il peut y a voir intérêt à extraire au moins une partie de la phase liquide de la phase solide en vue de la valoriser et d'optimiser le procédé global.

Le brevet WO 2014/135755 propose un procédé de traitement de biomasse avec la succession d'étapes suivantes : a- on effectue une étape de prétraitement par mise en contact et chauffage de la charge de biomasse avec de l'eau et un composé acide ou basique, de manière à obtenir un substrat prétraité, b- on met en contact le substrat prétraité avec des enzymes cellulases et avec un flux liquide enrichi en produits de fermentation obtenu à l'étape e) de manière à obtenir un hydrolysat comportant un résidu solide et une phase liquide contenant des sucres, c- on effectue une fermentation alcoolique de l'hydrolysat au moyen d'un microorganisme alcooligène de manière à produire un vin de fermentation comportant une matière solide et une phase liquide contenant des produits de fermentation, d- on extrait au moins une partie de la matière solide contenue dans le vin de fermentation de manière à obtenir un flux enrichi en matière solide et un vin de fermentation appauvri en matière solide, e- on lave le flux enrichi en matière solide avec un flux liquide de manière à obtenir ledit flux liquide enrichi en produits de fermentation, le flux liquide enrichi en produits de fermentation étant recyclé à l'étape b), f- on effectue une étape de séparation du vin de fermentation appauvri en matière solide de manière à obtenir au moins un flux purifié comportant un alcool ou un solvant et au moins un flux de vinasse.

Ce brevet propose ainsi d'effectuer la séparation de la lignine et d'autres éventuels solides inertes après l'étape de fermentation. La matière solide composée majoritairement de lignine est ensuite soumis à un lavage pour récupérer les produits de fermentation piégés, en particulier les alcools et les solvants. Le liquide de lavage est ensuite recyclé dans l'unité d'hydrolyse enzymatique, qui peut être la même unité que l'unité de fermentation ou qui peut être distincte de l'unité de fermentation afin de ne pas apporter de la dilution aux flux existants.

Le brevet EP 2 774 992 propose d'effectuer, dans un procédé comportant une étape de prétraitement, une étape d'hydrolyse enzymatique puis une étape de fermentation, d'extraire au moins une partie du résidu solide contenu dans l'hydrolysat de manière à obtenir un flux de résidu solide comportant de la lignine et un hydrolysat appauvri en résidu solide, puis le flux de résidu solide est lavé avec un flux liquide de façon à récupérer un flux liquide enrichi en sucres, qui peut être recyclé à l'étape d'hydrolyse enzymatique pour pouvoir valoriser les sucres sans apporter de dilution des flux dans le procédé.

Dans ces deux brevets, on réalise donc des lavages et extractions de produits intermédiaires, visant à séparer à partir d'un mélange solide/liquide une phase enrichie en matière solide et une phase enrichie en liquide, en utilisant des dispositifs conventionnels comme un dispositif de décantation ou de percolation pour réaliser l'extraction/la séparation et un dispositif de lavage par contre-courant.

Cependant, réaliser une telle séparation solide/liquide est une opération délicate à standardiser, dans la mesure où le mélange peut présenter des caractéristiques très différentes, à la fois, notamment, selon sa place dans l'enchainement d'étapes du procédé global de traitement de la biomasse, selon le type de biomasse utilisée etc... Ces caractéristiques sont notamment le taux de matière sèche de la biomasse initiale ou encore la rhéologie du mélange à traiter. Le but de la séparation peut aussi différer, selon le point jusqu'où l'on veut pousser la séparation. Les dispositifs conventionnels n'ont pas nécessairement toute la flexibilité et la performance requises pour assurer des séparations sur des mélanges aussi différents.

L'invention a alors pour but de concevoir une nouvelle façon de réaliser des séparations solide/liquide à partir d'un mélange, qui soit améliorée par rapport aux technologies conventionnelles, notamment dans le contexte de procédés de traitement de biomasse. Elle a notamment pour but de concevoir des technologies plus performantes et/ou plus flexibles selon le mélange à traiter.

Optionnellement, l'invention a pour but de concevoir ce type de technologie qui, en outre, puisse être mise en oeuvre avec des dispositifs aussi compacts que possible.

### Résumé de l'invention

L'invention a tout d'abord pour objet un procédé de traitement d'une biomasse lignocellulosique, ledit procédé comprenant au moins l'étape suivante :
**a.** Prétraitement de la biomasse par au moins une opération de cuisson ou d'explosion à la vapeur, afin d'obtenir un substrat prétraité.

Ce procédé comprend également au moins une étape de :
**b.** Séparation liquide/solide sur au moins une partie du substrat prétraité obtenu à l'issue de l'étape **a** ou à l'issue d'une étape additionnelle de traitement dudit substrat après l'étape **a,** ladite étape de séparation comprenant deux sous-étapes successives :
- une sous-étape amont de contactage **b1** du mélange solide/liquide mise en oeuvre par un mélangeur en continu utilisant un fluide de mélange, éventuellement précédé d'une pompe, et
- une sous-étape aval **b2** d'extraction/lavage mise en oeuvre par un filtre en continu, notamment de type filtre à bandes, utilisant un fluide de lavage, pour obtenir une phase solide enrichie en solide et une pluralité de phases liquides enrichies en liquide, avec un fonctionnement du filtre de préférence en contre-courant entre la circulation du mélange solide/liquide à séparer et le fluide d'extraction/lavage, et un recyclage au moins partiel d'une phase liquide extraite du filtre à bandes en entrée du mélangeur en tant que fluide de mélange.

Dans tout le présent texte, les termes « amont », « aval », « suivant » ou « précédent » sont faits en référence au sens d'écoulement général du produit à traiter en question, de la partie solide de la biomasse à traiter pour être plus précis, dans l'installation mettant en oeuvre le procédé de l'invention.

Pour effectuer la séparation solide/liquide, l'invention propose ainsi de coupler deux opérations avec deux dispositifs spécifiques à chacune d'elles, à savoir d'abord un mélangeur pour mettre en contact le mélange à séparer avec un fluide de mélange (aqueux par exemple), qui peut fonctionner en continu, et qui alimente ensuite un filtre à bande pour opérer la séparation à proprement dite. Le mélangeur donne au mélange les caractéristiques appropriées (propriétés rhéologiques, taux de matière sèche...) pour pouvoir ensuite être traité par le filtre en continu Les deux dispositifs peuvent être montés en série, en fonctionnant tous les deux en continu, ce qui est nettement plus avantageux qu'un fonctionnement par batch. Pour le filtre, le terme « continu » se comprend pour le solide à traiter.

Le mélangeur en continu est connu dans son principe : il s'agit, schématiquement, d'un mélangeur comprenant un corps creux cylindrique avec une entrée alimentée avec le mélange, une vis intérieure de convoyage du mélange depuis l'entrée jusqu'à la sortie du mélangeur, et un circuit de fluide de mélange (de l'eau) mettant en contact le mélange dans le mélangeur avec le fluide en question. Le mélangeur peut être muni en entrée d'un doseur. Ce type de mélangeur continu est notamment commercialisé par la société PARIMIX sous l'appellation « mélangeur continu PARIMIX IMR ». Il présente notamment l'avantage de pouvoir opérer le contactage d'un mélange, même à fort débit, avec un volume de chambre de mélange faible, ce qui rend le dispositif très compact, ce qui est tout particulièrement recherché dans le cadre de l'invention. Il permet aussi un temps de séjour court du mélange dans le mélangeur, tout en permettant d'obtenir en sortie du mélangeur un produit très homogène et au taux de matière sèche voulu.

Le filtre en continu préféré est un filtre à bande, également connu dans son principe : schématiquement, il permet d'effectuer l'extraction et le lavage d'un mélange solide/liquide en continu, par convoyage du mélange sur différentes zones successivement d'une bande en toile. L'extraction se fait sous vide partiel au travers de cette toile qui transporte le mélange, de zone en zone, et qui est poreuse pour pouvoir effectuer une filtration/extraction. Il peut être muni, dans sa partie la plus avale, d'une presse pour achever l'extraction du liquide. Ce type de filtre à bande est par exemple commercialisé par la société BHS sous l'appellation « Filtre à bande continue BFR ». Ce type d'outillage est connu sous le terme de filtre à bande continue avec extraction sous vide.

D'autres filtres en continu sont utilisables également, par exemple sous forme d'une presse à bande ou sous forme d'une pluralité de presses à bandes en série.

L'invention utilise donc en série ces deux dispositifs fonctionnant tous les deux en continu (le filtre à bande fonctionnant cependant, stricto sensu en séquentiel, comme cela sera détaillé plus loin), la sortie du mélangeur venant alimenter l'entrée du filtre (ce qui est nettement plus avantageux qu'un fonctionnement par batch), d'où un ensemble très compact et facile à intégrer dans une installation existante de traitement de biomasse.

Ce que l'invention a découvert, c'est que la combinaison de deux choix de paramètres de fonctionnement de ces dispositifs était la condition nécessaire pour que la séparation obtenue soit tout d'abord faisable industriellement, et pour qu'elle soit aussi robuste et performante :
- d'une part, le choix d'un fonctionnement d'un filtre en continu, notamment d'un filtre à bandes et de préférence fonctionnant en contre-courant entre la circulation du mélange solide/liquide à séparer et le fluide d'extraction/lavage (le fonctionnement en contre-courant s'étant avéré plus efficace qu'un fonctionnement en co-courant,
- d'autre part un recyclage au moins partiel d'une phase liquide extraite du filtre en entrée du mélangeur en tant que fluide de mélange, ce qui a permis de diminuer la consommation en fluide de mélange, généralement de l'eau, du mélangeur, et qui s'est avéré, de façon surprenante, permettre à la fois un mélange plus efficace au niveau du mélangeur et une extraction de phase(s) liquide(s) au niveau du filtre plus concentrée(s).

De préférence, on réalise la séparation du mélange solide/liquide lors de l'étape **b** sur une première partie du substrat prétraité obtenu à l'issue de l'étape **a**, et on soumet une seconde partie dudit substrat prétraité à au moins une étape ultérieure de traitement, notamment une hydrolyse puis une fermentation, et on soumet également la phase solide enrichie en solide obtenue à l'étape **b** à au moins une étape ultérieure de traitement, notamment la même ou les mêmes que celles subies par la seconde partie du substrat prétraité.

On peut ainsi extraire une partie du liquide du marc prétraité, l'ensemble du marc simplement prétraité et du marc prétraité puis séparé selon l'invention suivant ensuite le même déroulement d'étapes, et étant par exemple injectés dans le même réacteur de l'étape suivante pour y subir la même transformation.

L'intérêt est alors de pouvoir prélever juste une partie de la phase liquide (juste la quantité suffisante) sur une partie du marc prétraité, en vue d'une valorisation ou exploitation dans le procédé même de la phase liquide contenant des sucres (appelée aussi « jus sucré » dans le présent texte).

Avantageusement, selon un mode de réalisation, les phases liquides enrichies en liquide issues de l'étape de séparation **b** sont des jus sucrés, et ledit procédé comprend aussi une étape **c** de production d'enzymes et/ou une étape **d** de production de levures, et moins un desdits jus sucrés est utilisé pour ladite production d'enzymes **c** ou de levures **d** (propagation/croissance des micro-organismes). On peut ainsi, dans ce cas de figure, extraire juste la quantité nécessaire de jus sucré pour alimenter la production d'enzymes ou de levures, et limiter ainsi la séparation du marc prétraité au juste nécessaire.

Le procédé selon l'invention peut avantageusement comprendre une étape **e** d'hydrolyse enzymatique du substrat prétraité issu de l'étape **a** de prétraitement pour obtenir un hydrolysat, et on peut réaliser alors la séparation solide/liquide **b** sur au moins une partie dudit hydrolysat, notamment sur tout ledit hydrolysat.

Selon un mode de réalisation, on réalise la séparation solide/liquide **b** sur au moins une partie, notamment tout, du substrat prétraité obtenu à l'issue de l'étape **a** de prétraitement pour obtenir une phase solide enrichie en solide, et le procédé comprend aussi une étape **e** d'hydrolyse enzymatique de ladite phase solide enrichie en solide pour obtenir un hydrolysat. Dans ce cas de figure, on préfère donc séparer tout le marc prétraité et exploiter toute la phase liquide extraite, ce qui est intéressant quand on vise expressément la production de jus sucré pour le valoriser séparément du reste du procédé.

Avantageusement, le procédé selon l'invention comprend également les étapes suivantes après l'étape **a** de prétraitement :
**e.** Hydrolyse enzymatique du substrat prétraité, afin d'obtenir un hydrolysat comportant une matière solide et une phase liquide contenant des sucres,
**f.** Fermentation de l'hydrolysat, afin d'obtenir un vin de fermentation,
   l'étape **f** de fermentation pouvant succéder ou être concomitante à l'étape **e** d'hydrolyse enzymatique
**g.** Séparation/Distillation du vin de fermentation, afin d'obtenir un produit de distillation sous forme d'un alcool, d'un solvant, ou autre molécule biosourcée, et des vinasses,
l'étape **b** de séparation pouvant être réalisée sur au moins une partie, notamment l'ensemble, des produits obtenus après l'étape **e** d'hydrolyse et/ou **f** de fermentation et/ou **g** de séparation/distillation.

De manière optionnelle, dans l'étape **b** de séparation, on chauffe l'intérieur du mélangeur à une température d'au moins 30°C en fonctionnement, notamment comprise entre 40 et 60°C, notamment par des moyens de chauffage électriques intégrés. Il s'est en effet avéré que le chauffage du mélange pendant son transport à l'intérieur du mélangeur améliorait l'homogénéité du mélange en sortie du mélangeur. Les températures restent suffisamment peu élevées pour ne pas générer une trop forte consommation en énergie.

De préférence encore, dans l'étape **b** de séparation, on chauffe le fluide d'extraction/lavage avant introduction dans le filtre et/ou la phase liquide extraite du filtre et recyclée avant son introduction dans le mélangeur, notamment à une température d'au moins 30 °C, et d'au plus 90°C , notamment une température comprise entre 40°C et 80°C. Réchauffer la phase liquide avant son recyclage dans le mélangeur permet à celle-ci de participer à l'effort de chauffage du mélange dans le mélangeur. On peut alors régler de façon appropriée le chauffage obtenu par les moyens de chauffage équipant le mélangeur et le chauffage obtenu par la circulation dans le mélangeur d'un fluide de mélange chaud. Naturellement, on peut aussi chauffer le fluide de mélange ne provenant pas du recyclage depuis le filtre (généralement de l'eau), en plus ou à la place du chauffage de la phase liquide recyclée depuis le filtre.

Avantageusement, dans l'étape **b**, le filtre est un filtre à bande qui comprend au moins deux, notamment au moins trois, zones successives, avec soutirage dans au moins une, notamment dans chaque, zone d'une phase liquide. Les filtres à bande sont très souples de mise en oeuvre : on peut prévoir le nombre et la dimension, notamment la longueur de zones que l'on veut, on peut regrouper des zones pour ne soutirer qu'une seule phase liquide par groupement de zones, les zones peuvent avoir des longueurs différentes, par exemple des longueurs croissantes ou décroissantes selon l'axe de transport du mélange sur la bande....

De préférence, la phase liquide de la première et/ou de la deuxième zone est recyclée au moins en partie en entrée du mélangeur de l'étape **b.** On préfère donc recycler la ou les phases liquides extraites le plus amont en du filtre à bande, c'est-à-dire celles qui sont les plus concentrées, ce qui permet d'obtenir en sortie de mélangeur un liquide plus concentré. De préférence, la phase liquide soutirée de la troisième et/ou des zones suivantes et/ou de la dernière zone est recyclée au moins en partie en tant que fluide de lavage/extraction du filtre à bande : c'est la façon dont on met en oeuvre le contre-courant dans le fonctionnement du filtre à bande dans l'invention.

Optionnellement, dans l'étape **b**, on peut équiper le filtre, notamment à bande, d'une presse dans sa partie terminale. On améliore ainsi encore la séparation.

Avantageusement, on peut sélectionner les paramètres de fonctionnement des étapes **b1** et **b2** de séparation en fonction des caractéristiques du mélange solide/liquide, pour que les phases liquides produites extraites du filtre à bande aient une concentration d'au moins 30g de produit/kg, notamment au moins 35 ou 40, et de préférence d'au moins 50g de produit /kg, notamment au moins 50g de sucre/kg quand la séparation **b** est réalisée sur le substrat prétraité issu de l'étape **a** de prétraitement. C'est en effet à partir d'une telle concentration que les jus sucrés peuvent être efficacement valorisés en tant que tels.

Les paramètres de fonctionnement préférés pour atteindre ce résultat sont notamment le taux de recyclage de la phase liquide extraite du filtre en entrée du mélangeur, ou encore la configuration du recyclage (à savoir un seul type de jus recyclé ou plusieurs jus issus de plusieurs zones du filtre notamment, qui auront ainsi des concentrations en sucre différentes, selon des proportions ajustables, et/ou un ajout d'eau en quantité ajustable à ce ou ces jus recyclés au niveau du mélangeur).

L'invention a également pour objet une installation de traitement d'une biomasse lignocellulosique, notamment destinée à mettre en oeuvre le procédé précédemment décrit, et qui comprend au moins :
- une zone de prétraitement de la biomasse par au moins une opération de cuisson ou d'explosion à la vapeur, afin d'obtenir un substrat prétraité,
- une zone de séparation liquide/solide sur au moins une partie du substrat prétraité obtenu en sortie de la zone de prétraitement ou d'une zone additionnelle de traitement dudit substrat après la zone de prétraitement, ladite zone de séparation comprenant deux sous-zones en série: - une sous-zone amont de contactage comprenant un mélangeur en continu utilisant un fluide de mélange, éventuellement associé à une pompe amont, et - une sous-zone aval d'extraction/lavage comprenant un filtre en continu, notamment de type filtre à bandes avec un fluide de lavage, avec un fonctionnement du filtre de préférence en contre-courant entre la circulation du mélange solide/liquide à séparer et le fluide d'extraction/lavage, et un recyclage au moins partiel d'une phase liquide extraite par le filtre à bandes en entrée du mélangeur en tant que fluide de mélange.

L'installation peut prévoir, dans la zone de séparation : - des moyens de connexion fluidique entre le filtre à bande et le mélangeur pour recycler au moins une partie d'une phase liquide extraite par le filtre en entrée du mélangeur et - des moyens de connexion fluidique pour recycler une autre phase liquide extraite du filtre en tant que fluide de lavage dudit filtre.

Le filtre est de préférence un filtre à bandes, qui peut être optionnellement équipé d'une presse dans sa partie terminale.

L'installation de traitement peut comprendre également une zone de production d'enzymes et/ou une zone de production de levures, et prévoir un moyen de transfert d'au moins une phase liquide extraite du filtre à bande depuis la zone de séparation à la zone de production d'enzymes et/ou à la zone de production de levures, ladite phase liquide étant un jus sucré.

L'invention a aussi pour objet l'utilisation du procédé ou de l'installation décrits plus haut pour le traitement de biomasses du type bois, paille, résidus agricoles, et toutes cultures énergétiques dédiées, notamment des plantes annuelles ou pluriannuelles telles que le miscanthus en vue de produire des sucres, des biocarburants ou des molécules biosourcées. De façon plus générale, la biomasse lignocellulosique, ou matériaux lignocellulosiques employés dans le procédé selon l'invention est obtenue par exemple à partir de bois (feuillus et résineux), brut ou traité, de sous-produits de l'agriculture tels que la paille, de fibres de plantes, de cultures forestières, de résidus de plantes alcooligènes, sucrières et céréalières, de résidus de l'industrie papetière, de biomasse marine (par exemple macroalgues cellulosiques) ou de produits de transformations de matériaux cellulosiques ou lignocellulosiques. Les matériaux lignocellulosiques peuvent également être des biopolymères et sont préférentiellement riches en cellulose.

L'invention permet de produire comme produits valorisables à la fois du biocarburant comme de l'éthanol et des jus sucrés, ou que du biocarburant, ou que des jus sucrés, avec une grande flexibilité.

### Description détaillée

L'invention sera ci-après détaillée à l'aide d'exemples non limitatifs illustrés par les figures suivantes :
- Figure 1 : une représentation synoptique des outillages utilisés dans le cadre de l'invention pour réaliser l'étape **b** de séparation d'un mélange solide/liquide dans un procédé de traitement de biomasse ;
- Figures 2a et 2b : deux exploitations différentes des outillages représentés à la figure 1, selon le type de biomasse utilisé ;
- Figure 3 : une représentation schématique (coupe transversale longitudinale) du mélangeur appartenant aux outillages représentés à la figure 1 ;
- Figure 4 : une représentation fonctionnelle sous forme de schéma bloc de l'étape de séparation **b** opérée par l'invention intégrée dans une étape de prétraitement de biomasse selon une première variante ;
- Figure 5 : une représentation fonctionnelle sous forme de schéma bloc d'un procédé complet de traitement de biomasse intégrant l'étape de séparation **b** opérée par l'invention selon la figure 4 ;
- Figures 6,7,8 : une représentation fonctionnelle sous forme de schéma bloc d'un procédé complet de traitement de biomasse intégrant l'étape de séparation **b** opérée par l'invention selon respectivement une deuxième, troisième et quatrième variante.

### Description des figures

Les mêmes références correspondant aux mêmes composants / fluides / produits sur l'ensemble des figures. Les figures sont très schématiques, ne sont pas nécessairement à l'échelle et ne représentent pas tous les composants de l'outillage, tous les détails des procédés concernés, mais seulement ceux intéressant plus particulièrement la description de l'invention.

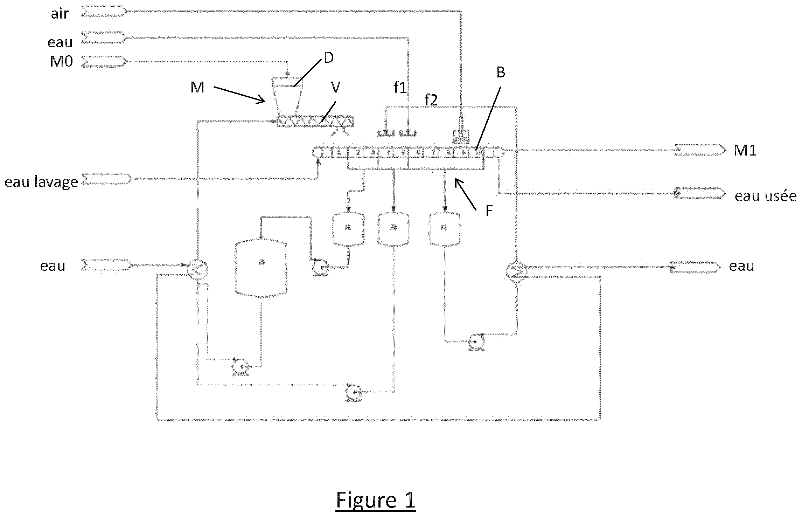

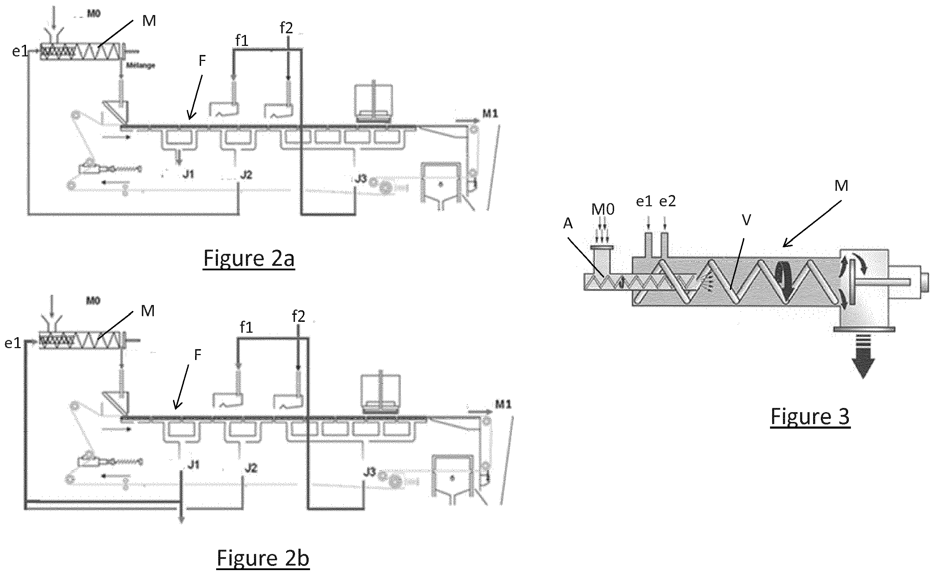

La figure 1 représente donc les outillages mis au point dans le cadre de l'invention pour effectuer la séparation solide/liquide d'un mélange dans un procédé de traitement de biomasse lignocellulosique : le mélange à séparer est d'abord traité par un mélangeur M en continu de type IMR de PARIMIX, représenté plus en détails à la figure 3, puis par un filtre à bande F du type filtre à bande continue sous vide, du type BFR de BHS. Ils sont ici tous les deux disposés substantiellement horizontalement, à des hauteurs différentes, le mélangeur étant disposé au-dessus du filtre à bande.

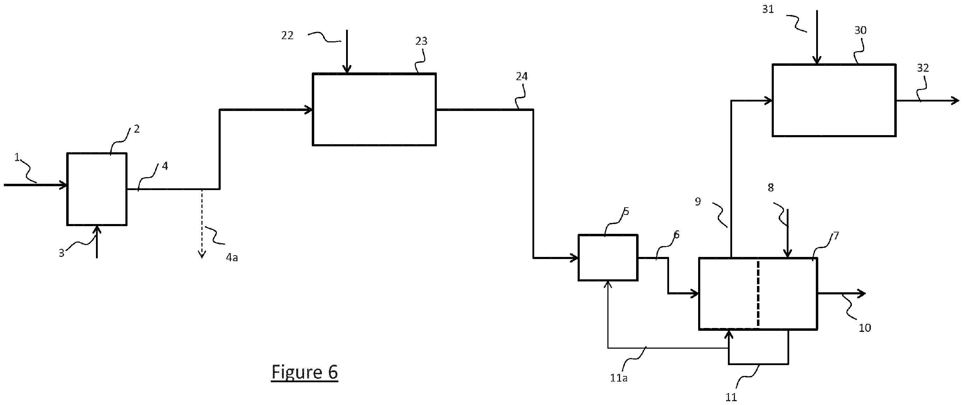

Pour simplifier la description de ces outillages, par soucis de concision, il sera considéré que le mélange à séparer est un marc prétraité d'un procédé de traitement de biomasse, bien que la séparation selon l'invention puisse s'appliquer à d'autres mélanges solides/liquide d'un tel procédé (comme décrit plus loin à l'aide des figures 6 et suivantes).

Le mélangeur M permet un repulpage des marcs prétraités à haute teneur en matière sèche (MS) et en solides, par ajout d'un fluide de mélange et par un malaxage obtenu par la spire sans fin du mélangeur convoyant le marc d'une extrémité à l'autre du mélangeur. Le mélangeur M, comme détaillé à la figure 3, comprend un corps cylindrique muni d'une vis V tournante sans âme, avec son extrémité amont muni d'un doseur D dosant en continu le mélange à séparer, qui est ensuite injecté dans le mélangeur par un tube d'alimentation ou trémie A connecté au doseur. Il est représenté en position de fonctionnement, c'est-à-dire selon un plan sensiblement horizontal.

Dans sa partie amont, le corps cylindrique du mélangeur est muni d'entrée(s) de fluide(s) de mélange e1, e2, le ou les fluides sont centrifugés par les spires, le vortex liquide ainsi créé rencontre le flux de marc éclaté en sens inverse. Grâce à ce principe, le volume de la chambre de mélange de ce mélangeur est faible, la puissance utilisée est également faible et le mélangeur dans son ensemble est compact. Ici, le mélangeur est équipé de moyens de chauffage électriques (non représentés) pour chauffer l'intérieur du corps cylindrique à une température d'environ 40 à 50°C, cet apport de chaleur favorisant l'obtention d'une plus grande homogénéité du mélange en sortie du mélangeur à temps de séjour constant dans celui-ci. Le marc repulpé, en sortie du mélangeur M, tombe par gravité sur la partie amont de la bande du filtre à bande F.

La séparation de ce marc sur le filtre à bande F avec lavage à contre-courant permet d'extraire des jus concentrés en sucres. La technologie du filtre à bande F est basée sur la filtration sous vide, dont le principe est de répartir le marc prétraité sur une bande avançant de manière séquentielle. Cette bande poreuse permet, par tirage sous vide, de séparer les liquides (les jus) du solide en formant un gâteau (le marc lavé), qui tombe en fin de bande. Du fluide de lavage est projeté au-dessus de la bande pour laver le gâteau qui se forme progressivement au long de la bande. Les jus sont partiellement recyclés, comme détaillé plus loin. Ils peuvent aussi être regroupés en un seul flux.

Le filtre à bande F possède dans cet exemple 10 zones actives de vide, numérotées 1 à 10 sur la figure 1, où peuvent avoir lieu différentes étapes du procédé de lavage et de filtration (zones déplaçables et redimensionnables dans le filtre). Il peut être équipé de bandes de polymère du type polyuréthane ou silicone sur le côté de la bande filtrante pour limiter les pertes de vide et améliorer la séparation. On peut prévoir d'ajouter une cloison en partie haute (ou tout autre moyen mécanique équivalent), juste après la ou les premières zones, pour permettre une meilleure répartition du mélange sur la bande.

On décrit maintenant les différents flux entrant et sortant de ces appareillages, depuis l'amont vers l'aval : le marc prétraité M0 entre dans le doseur D du mélangeur M, tandis que le fluide de mélange est introduit dans le mélangeur par les entrées e1, e2 (représentées qu'à la figure 3). Le marc mélangé M1 (désigné aussi par « MoRe » plus loin) sort à l'extrémité aval du mélangeur M et tombe par gravité sur la bande B du filtre F dans sa partie amont. La bande convoie le marc jusqu'à son extrémité aval, avec extraction sous vide, sous la bande, de trois phases liquides J1, J2 et J3, qui sont des jus sucrés de concentrations décroissantes. Au-dessus de la bande, dans sa partie amont ou sa partie centrale, sont introduits deux fluides f1 et f2 d'extraction/lavage par des buses à distance l'une de l'autre. Le fluide f1 peut être de l'eau, le fluide f2 est le recyclage partiel ou total du jus J3.

Il reste naturellement dans le cadre de l'invention d'extraire sous vide non pas trois phases liquides, mais davantage, au plus le même nombre que de zones dans le filtre à bandes.

Le déplacement du fluide d'extraction/ lavage se fait à contre-courant de la circulation du mélange sur la bande. Dans le cadre de la présente invention, c'est en fait, de manière connue, un contre-courant « simulé », en ce sens qu'il n'y a pas d'écoulement à contre-courant à proprement parlé dans chacune des zones de lavage. Le principe du filtre à bande repose sur un lavage tangentiel : le mélange se déplace ici latéralement, sur la figure à titre d'exemple de gauche à droite, et le liquide de lavage (appelé plus haut fluide d'extraction/lavage de façon plus générale) se déplace du haut vers le bas. L'injection de liquide sur le mélange qui se déplace sur la bande peut se faire, notamment, soit par déversement, c'est-à-dire par écoulement gravitaire, soit par aspersion... Le mouvement de haut en bas du liquide traversant le mélange se déplaçant sur la bande est imposé par le vide que l'on crée sur la bande. Le filtre a un fonctionnement séquentiel sur le mélange comprenant la phase solide à extraire (appelé aussi « gâteau »), que l'on peut schématiser ainsi : - temps 1, la bande avance, - temps 2, la bande s'arrête et le vide est créé (et le cas échéant, on presse) et du jus est extrait dans chacune des zones, et ainsi de suite. A noter, généralement, que l'injection des liquides de lavage est, elle, réalisée en continu (on injecte quand la bande avance et quand elle est à l'arrêt). Le contre-courant est « simulé » par la réinjection du jus extrait en amont de son extraction.

Tout ou partie d'au moins l'un des jus J1, J2, J3 peut être recyclé comme fluide de mélange dans le mélangeur M.

Sous les 10 zones de la bande poreuse du filtre F, on extrait donc sous vide partiel ici trois phases liquides notées J1, J2 et J3, tandis que la phase solide progressivement enrichie en solide, le « gâteau » G, poursuit sa course jusqu'en partie aval de la bande, d'où il est extrait après être soumis à une presse P disposée en toute fin de la bande, presse qui termine la séparation. Cette étape de pressage est optionnelle.

Le ou les fluides de lavage f1,f2 sont chauffés, par exemple à une température de 30 à 85°C avant d'être déversés sur le dessus du gâteau convoyé par la bande poreuse, à l'aide de moyens de chauffage équipant les conduits d'amenée.

Le flux du ou des jus sucrés qui est recyclé soit comme fluide de mélange dans le mélangeur soit comme fluide de lavage/extraction dans le filtre est/sont également chauffé(s) avant réintroduction dans le filtre ou dans le mélangeur, également à l'aide de moyens de chauffage équipant leurs conduits d'amenés, par exemple à une température de 30 à 85 ° C.

La figure 2a représente une première façon de mettre en oeuvre l'installation de la figure 1, pour séparer le marc prétraité d'une biomasse à base de Miscanthus : le jus J3 est recyclé entièrement dans l'entrée la plus amont, l'entrée f1, de fluide de lavage extraction du filtre F, le jus J2 est entièrement recyclé en entrée e1 du mélangeur M, en appoint à une entrée e2 de fluide mélangeur sous forme d'eau. La totalité du jus J1 est extraite pour utilisation/valorisation en dehors de cette installation de séparation.

La figure 2b est une autre façon de mettre en oeuvre l'installation de la figure 1, pour séparer le marc prétraité d'une biomasse à base de paille. La différence d'avec la figure 2a est qu'ici une partie du jus J1 aussi est recyclé en tant que fluide de mélange dans le mélangeur avec le jus J2.

La proportion de chacun des jus J1, J2, J3 ... extraits du filtre à bande qui est recyclée dans le mélangeur ou dans le filtre peut être très variable, depuis 0% jusqu'à 100%. Elle va dépendre de la nature de la biomasse (du taux de sa matière sèche), de la consommation en eau voulue, de la concentration voulue en agents actifs (en sucres ici) des jus extraits en fonction de leur utilisation ultérieure, dans un procédé global de traitement de biomasse, selon les besoins, ou comme produit de valorisation indépendant.

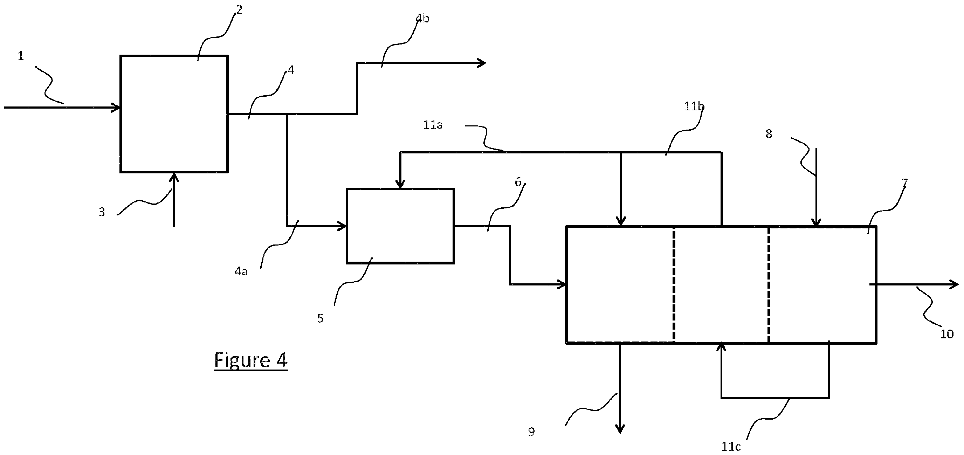

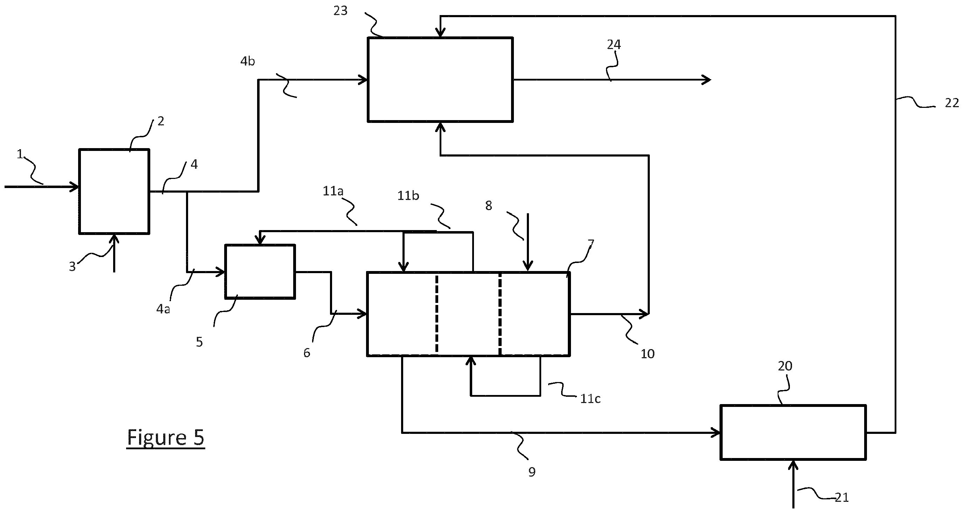

La figure 4 représente l'intégration de l'étape de séparation **b** dans un prétraitement de biomasse sous forme de schéma bloc. Les références représentées ont la signification suivante :
1. Biomasse à traiter
2. Etape de prétraitement
3. Flux de fluide nécessaire au prétraitement **a** de la biomasse (eau, catalyseur acide, vapeur, ...)
4. Flux de marc prétraité, séparé en deux flux 4a qui va vers l'étape de séparation **b** et 4b (qui va vers l'étape d'hydrolyse comme détaillé plus loin à l'aide de la figure 5)
5. Mélangeur utilisé dans l'étape b de séparation selon l'invention
6. Mélange en sortie du mélangeur
7. Séparateur filtre à bande utilisé dans l'étape **b** de séparation selon l'invention
8. Fluide de lavage (généralement de l'eau)
9. Jus sucré extrait
10. Solide lavé
11. Jus intermédiaires qui sont renvoyés au filtre à bande et dont au moins une partie (11a) est envoyée au mélangeur

Le bloc 2 est la première étape de conditionnement et prétraitement d'un flux entrant de biomasse 1 par un ou différents réactifs, par mise en contact avec ou plusieurs autres flux entrants 3 de fluide (eau, eau avec un catalyseur chimique de type acide, basique, oxydant, vapeur d'eau...) et éventuel(s) traitement(s) thermique(s). En flux sortant, on obtient un flux de marc prétraité 4.

Le bloc 5 opère donc la sous-étape b1 de la séparation b selon l'invention : c'est le mélangeur M qui reçoit en entrée une portion 4a du flux de marc prétraité 4 et au moins un flux de liquide de mélange comprenant une partie des jus 11a extrait du filtre à bande 7 en aval du mélangeur 5.

Le bloc 7 opère la sous-étape b2 de séparation avec le filtre à bande : il reçoit en entrée le flux de solide 6 qui sort du mélangeur du bloc 5, et au moins un flux de liquide de lavage 8, avec recyclage d'une partie 11c des jus extraits de l'aval vers l'amont du filtre et d'une autre partie 11a vers l'entrée du mélangeur du bloc 5, il en sort un flux de solide 10 lavé.

La figure 5 représente l'intégration de l'étape de séparation b selon l'invention dans le procédé complet de traitement de biomasse selon une première variante conforme à la figure 4. Après les blocs 2,5 et 7 déjà décrits, les nouvelles références ont la signification suivante :
20. Etape(s) de production de biocatalyseurs qui utilise le jus sucré 9
21. Flux de fluides nécessaires à la production de biocatalyseurs
22. Solution contenant les biocatalyseurs (enzymes et/ou levures), et éventuellement autres fluides non représentés
23. Etape d'hydrolyse enzymatique dans laquelle est introduit l'autre portion 4b du marc prétraité 4, et optionnellement le marc lavé 10 (ou étape d'hydrolyse enzymatique et fermentation simultanées SSF)
24. Mélange de solide non hydrolysé et de sucres issus de l'hydrolyse en solution (ou mélange de solide non-hydrolysé et de produit de la fermentation en cas de SSF)

Cette figure représente donc l'invention cette fois intégrée dans le procédé de traitement, qui, au-delà du prétraitement opéré sur la biomasse, se poursuit par l'hydrolyse enzymatique et la fermentation du marc.

Le marc prétraité 4 a été partagé en un flux 4a qui est séparé selon l'invention comme décrit à la figure précédente, et un flux 4b qui est dirigé vers l'étape 23 d'hydrolyse enzymatique. Optionnellement, le marc séparé et lavé 10 selon l'invention peut aussi être dirigé vers cette étape 23 avec le flux 4b. (II peut sinon être valorisé différemment, dans le procédé de traitement de biomasse ou ailleurs). Il en sort un flux 24 qui peut, selon que l'hydrolyse se fait simultanément avec la fermentation (SSF) ou pas, être composé d'un mélange de solide non hydrolysés et de sucres issus de l'hydrolyse (sans SSF) ou d'un mélange de solide non hydrolysé et de produits de fermentation (avec SSF).

Le bloc 20 est la ou les étapes de production des biocatalyseurs (enzymes, levures) qui utilise(nt) le jus sucré 9 issu de la séparation par le filtre à bande du bloc 7 et un ou des flux de fluides 21 nécessaires à la production des biocatalyseurs (eau, nutriment, autres sucres, produits chimiques par exemple pour réguler pH, etc...), ce jus sucré 9 servant alors de substrat de croissance/propagation ou de production aux microorganismes (champignons) produisant les enzymes et/ou aux levures.

Dans cette première variante, le jus extrait 9 est entièrement utilisé pour la production de levures et/ou d'enzymes. On peut aussi l'utiliser à ces fins que pour une partie seulement du flux 9.On peut aussi valoriser tout ou partie de ce jus sucré 9 indépendamment du procédé de traitement de biomasse (le valoriser en tant que tel par exemple).

On peut aussi faire subir à tout le marc prétraité l'opération de séparation selon l'invention, et non pas sur une partie seulement de celui-ci, puis envoyer l'ensemble du marc séparé dans le bloc suivant d'hydrolyse enzymatique/fermentation.

Il est également possible d'utiliser directement une partie du marc brut (c'est-à-dire en sortie de l'étape de prétraitement) pour la production des enzymes et/ou la propagation de levures. Pour la propagation de levures utilisant un marc issu du prétraitement de biomasse, on peut se rapporter par exemple au brevet WO 2016/193576. Pour la production d'enzymes utilisant un marc prétraité, on peut par exemple se reporter au brevet WO 2017/174378.

Il sort du bloc 20 un flux 22 contenant les biocatalyseurs (enzymes et/ou levures), et éventuellement d'autres fluides (par exemple: eau, produits chimiques notamment pour réguler le pH ...), biocatalyseurs si produits autrement que sur jus sucré, nutriments, etc...), ce flux 22 sortant étant injecté en entré du bloc 23 d'hydrolyse enzymatique.

Une fois la production d'enzymes réalisée, soit on exploite tout le moût, c'est-à-dire à la fois les enzymes et les champignons qui les ont produites, soit uniquement les enzymes (on doit alors faire une étape de séparation /purification préalable), pour réaliser l'hydrolyse enzymatique de la biomasse prétraitée.

De préférence, le cocktail enzymatique a été produit par un champignon *Trichoderma reesei.*

En ce qui concerne la production des levures, la levure produite est de préférence une levure de type *Saccharomyces cerevisiae* génétiquement modifiée pour consommer le xylose. Le flux obtenu qui va être exploité dans l'étape de fermentation peut contenir les levures en mout entier ou concentré, dans ce dernier cas on parle de crème de levures (on doit alors effectuer une étape de concentration préalable).

La figure 6 propose d'intégrer dans une deuxième variante l'étape de séparation b de l'invention. Les nouvelles références ont la signification suivante :
30. Etape de fermentation, par exemple en éthanol
31. Flux de fluides nécessaires à la fermentation
32. Solution contenant le produit de la fermentation

Ici, on a donc un bloc 30 d'étape de fermentation distinct de l'étape 23 d'hydrolyse enzymatique, et la séparation des blocs 5,7 de l'invention s'insère entre ces deux étapes : Le flux 24 issu de l'étape d'hydrolyse enzymatique est séparé selon l'invention, et le flux 9 en sortie du bloc 7 opérant la séparation par le filtre à bande est envoyé en flux entrant dans le bloc 30 de fermentation, également alimenté en flux entrant 31 du ou des fluides nécessaires à la fermentation, comme des biocatalyseurs (levures), et éventuellement des nutriments etc...

Le flux 32 sortant de l'étape de fermentation contient le produit de fermentation, qui peut ensuite subir des étapes conventionnelles de séparation, du type distillation, déshydratation, séparation solide/liquide (non représentées) qui permettent d'obtenir la molécule biosourcées voulue (ici de l'éthanol comme biocarburant), des résidus solides et des résidus liquides appelés également vinasses.

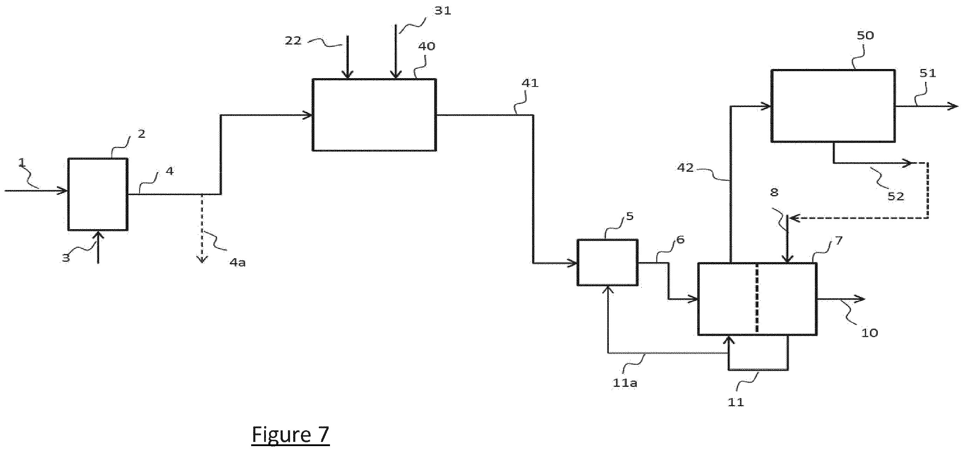

La figure 7 propose d'intégrer dans une troisième variante l'étape de séparation b de l'invention. Les nouvelles références ont la signification suivante :
40. étapes d'hydrolyse et fermentation
41. Flux contenant le solide non hydrolysé et le produit de fermentation
42. flux de liquide solution contenant le produit de fermentation
50. Etape de séparation du produit de fermentation
51. Flux de produit de fermentation purifié
52. flux de vinasses

Le bloc 40 représente donc l'étape d'hydrolyse enzymatique et l'étape de fermentation qui peuvent être séparées ou simultanées, représentées par un bloc commun par soucis de clarté. Le flux 41 issu de ces deux étapes et qui contient donc une partie de solide non hydrolysé et le produit de fermentation est amené en entrée des blocs 5,7 de séparation selon l'invention. Le flux de liquide contenant des produits de fermentation sortant 42 de cette séparation alimente le bloc 50 de l'étape de séparation des produits de fermentation (ce flux de liquide est donc de composition différente du flux 9 des variantes précédentes, qui était seulement un jus sucré). En sortie du bloc 50, on récupère un flux de liquide 51 de produit de fermentation partiellement purifié, et un flux 52 de vinasses, qui peut optionnellement (flèches en pointillés sur la figure) être recyclé en remplacement de l'eau de lavage 8 du filtre à bande du bloc 7.

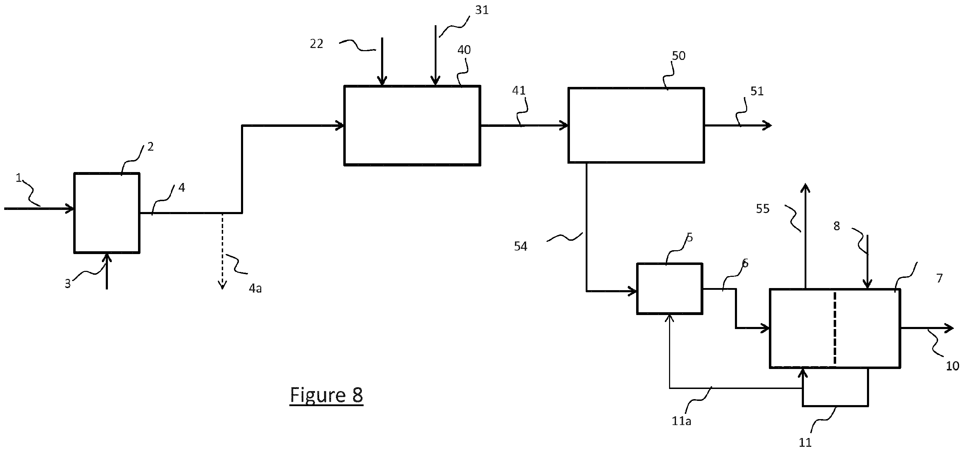

La figure 8 propose d'intégrer dans une quatrième variante l'étape de séparation b de l'invention. Les nouvelles références ont la signification suivante :
54. milieu séparé du produit contenant le solide non-hydrolysé et du liquide
55. vinasses

Ici, l'étape b de séparation de l'invention est opérée après l'étape de séparation 50 : le flux 54 issu de la séparation 50 et comportant un milieu séparé du produit contenant le solide non hydrolysé et du liquide est envoyé dans le mélangeur 5, le flux de solide 55 sortant du filtre à bande du bloc 7 sont des vinasses extraites par lavage donc. Dans cette variante, l'étape 50 de séparation peut être couplée à l'étape de fermentation, on parle de couplage fermentation/séparation, soit in situ, dans le réacteur de fermentation même, soit sur un autre circuit à part, où le milieu après séparation retourne en fermentation.

Dans toutes ces variantes, on voit qu'on peut insérer l'étape de séparation b selon l'invention entre des étapes connues d'un procédé de traitement de biomasse, ici pour exploiter les jus sucrés comme substrats pour la propagation, croissance ou la production d'enzymes de micro-organismes nécessaires à la conversion de la biomasse, mais aussi quand on veut valoriser en tant que tels ces jus sucrés.

### Exemples de réalisation

Ils utilisent les schémas de procédé et les outillages détaillés à l'aide des figures précédentes.

Cinq exemples vont être décrits, sur trois types de biomasses différents : exemple 1 sur TCR (Taillis à Croissance Rapide), exemple 2 sur Miscanthus, exemple 3 sur paille, exemple 4 sur une autre configuration de paille, et exemple 5 sur une autre configuration sur TCR.

L'objectif est d'obtenir des jus sucrés J1 avec une concentration en sucres de 50 g/kg minimum.

Dans les exemples décrits ci-après, l'acronyme "MS" désigne le taux de matière sèche qui est mesurée selon la norme ASTM E1756 - 08(2015) « Standard Test Method for Détermination of Total Solids in Biomass".

Les conditions opératoires sont les suivantes :
Le mélangeur M est un mélangeur continu PARIMIX IMR, qui permet un repulpage des marcs à hautes teneurs en matières sèche (MS) et solides. Il est chauffé par des moyens électriques à environ 40-50°C.

La séparation sur le filtre à bande F, de type filtre à bande en continu BFR de la société BHS avec lavage à contre-courant, permet d'extraire des jus concentrés en sucres. La technologie du filtre à bande est basée sur la filtration sous vide. Le principe est de répartir le marc prétraité sur une bande avançant de manière séquentielle. Cette bande poreuse permet, par tirage sous vide, de séparer les liquides (les jus) du solide en formant un gâteau (le marc lavé), qui tombe en fin de bande. Les jus sont partiellement recyclés.

Le filtre F utilisé possède 10 zones actives de vide, où peuvent avoir lieu différentes étapes du procédé de lavage et de filtration (zones déplaçables dans le filtre). Les zones sont regroupées en trois groupes de zones, qui sont modulables, et qui permettent d'extraire trois jus J1, J2 et J3. Le filtre est muni, sur les côtés de la bande poreuse, de bandes en polyuréthane ou en silicone, pour limiter les pertes au vide observées et pour améliorer la filtration du produit. Le filtre est muni d'une paroi verticale au-dessus de la bande juste après la zone d'alimentation en mélange pour permettre une meilleure distribution du mélange sur le filtre.

Pour augmenter la récupération des sucres solubles et réduire le temps de filtration, les flux des jus recyclés J2 (et J1 le cas échéant), J3 ainsi que l'eau de lavage F1 sont chauffés :
- Le jus J3 est chauffé et recyclé vers la première buse d'eau de lavage f1
- L'alimentation en eau de lavage chaude se faisait par la seconde buse de lavage f2
- Le jus J2 est chauffé et recyclé jusqu'au repulpage du marc prétraité en entrée du mélangeur (et le jus J1 le cas échéant)

Pour la paille (exemple 3 et 4), ainsi que pour un exemple concernant le peuplier TCR (exemple 5), une partie du jus J1 a été aussi recyclée vers le mélangeur M pour augmenter la concentration en sucres dans le jus J1.

Les caractéristiques du filtre et les conditions opératoires testées sont les suivantes :
- Référence : BF025-020,
- Toile : 058000-W120 (Polypropylène 120µm; 05-8000-S120)
- Matériau : polymère
- Débit : 180 kg/h de suspension traitable
- Surface filtration : 0,5 m²
- Niveau de vide : entre - 0,5 et -1 bar

Le tableau 1 ci-dessous regroupe les caractéristiques de teneur en solide et de teneur en matière sèche MS de la charge (biomasse prétraitée M0) qui est séparée selon l'invention :

**Tableau 1**

| | **Exemple 5 : TCR (2)** | **Exemple 4 : Paille (2)** | **Exemple 3 : Paille** | **Exemple 2** : **Miscanthus** | **Exemple 1 : TCR** |
|---|---|---|---|---|---|
| **Biomasse Prétraitée M0** | 44% solide | 29 - 32% solide | 29 - 30% solide | 42% solide | 38 - 43% solide |
| | 53 % MS | 41 - 46 % MS | 42 - 44 % MS | 54 % MS | 49 - 53% MS |

La vitesse de rotation de la vis d'alimentation du mélangeur M, et donc le débit du marc prétraité M0, est définie par la fréquence du doseur D. Cette vitesse influe sur le mélange et la consistance du marc repulpé en sortie du mélangeur. Une trop grande vitesse de la vis de mélange aboutit à un marc repulpé trop visqueux pour la suite du procédé. On prévoit généralement une trémie peseuse sur l'installation (non représentée) et un moyen de mesure du débit de marc, et on peut réguler l'alimentation en asservissant la vitesse de la vis à cette mesure de débit. On peut aussi choisir de fonctionner à vitesse fixe de la vis, qui aura été préalablement calibrée.

Le tableau 2 ci-dessous présente les débits de marc brut et de jus recyclé en entrée du mélangeur :

**Tableau 2**

| | **Débit marc brut en entrée (kg/h)** | **Jus recyclé (kg/h)** |
|---|---|---|
| **TCR (2) - exemple 5** | 50 | 130 |
| **Paille (2) - exemple 4** | 50 | 130 |
| **Paille - exemple 3** | 50 | 129 |
| **Miscanthus - exemple 2** | 70 | 95 |
| **TCR - exemple 1** | 50 | 75 |

### Exemple 1 selon l'invention : Biomasse à base de taillis à croissance rapide (TCR)

Les essais ont commencé avec un TCR, considéré souvent comme une matière première difficile à traiter. Pour améliorer l'extraction, les buses par aspersion ont été installées, un vide de - 0.6 bar a été réalisé, le bilan matière global et les conditions opératoires sont détaillés dans le tableau 3 ci-dessus.

**Tableau 3**

| | **Unité** | **TCR (2)** | **Paille (2)** | **TCR** | **Paille** | **Miscanthus** |
|---|---|---|---|---|---|---|
| Débit M0 | kg/h | 50 | 50 | 50 | 50 | 70 |
| Débit J2 recyclé | kg/h | 110 | 110 | 75 | 110 | 90 |
| Débit J1 recyclé | kg/h | 20 | 20 | 0 | 20 | 0 |
| Débit sortie du mélangeur M | kg/h | 180 | 180 | 125 | 180 | 160 |
| Temps d'avancement | s | 19 | 17 | 19 | 17 | 15 |
| Pression vide | bar | -0.6 | -0.45 | -0.6 | -0.45 | -0.15 |
| Pression pressage | bar | - | - | 3.3 | 3.7 | 2.6 |
| Température J1 | °C | 40 | 45 | 39 | 45 | 32 |
| Débit | kg/h | 67 | 50 | 45 | 70 | 55 |
| Température J2 après échangeur | °C | 80 | 80 | 75 | 75 | 75 |
| Débit J2 produit | kg/h | 75 | 110 | 75 | 115 | 90 |
| Température J3 après échangeur | °C | 80 | 80 | 75 | 75 | 75 |
| Débit J3 | kg/h | 75 | 105 | 60 | 105 | 80 |
| Débit d'eau lavage | kg/h | 70 | 69 | 55 | 83 | 55 |
| Température l'eau lavage | °C | 50 | 50 | 50 | 50 | 50 |
| Température M1 | °C | 26 | 35 | 40 | 35 | 45 |
| Débit M1 | kg/h | 60 | 65 | 60 | 52 | 92 |

### Exemple 2 selon l'invention : Biomasse à base de Miscanthus

L'extraction de jus sur le marc prétraité issu de Miscanthus n'a pas posé de problème. Le lavage (appelé aussi mélange, fait par le mélangeur M) est très efficace et des jus avec une concentration des sucres comprise entre 58-60 g/kg ont été produits. Le bilan matière global et les conditions opératoires sont détaillés dans le tableau précédent, et dans le tableau 4 suivant.

Le filtre à bande F a été alimenté avec 160 kg/h MoRe (18% MS) à 58°C. Deux buses de lavage par déversement ont été installées sur les zones 4 et 6 des 10 zones actives de la bande. Pour avoir l'espace le plus grand possible entre les flux, la première buse a lavé en mode contre-courant de la direction d'avancement du filtre, et la deuxième en mode co-courant. 45 kg/h de jus J1 à 65-68 g/kg de sucres a été produit pendant les deux premiers jours. Ensuite, le troisième jour, le débit d'eau de lavage a été augmenté pour produire plus de jus avec une concentration un peu moins importante. En conséquence, 55 kg/h de J1 a été produit avec une concentration de sucres comprise entre 58 et 60 g/kg.

### Exemple 3 selon l'invention : Biomasse à base de paille

Le premier essai sur la paille a été fait avec la même configuration que pour les deux exemples précédents. En conséquence, le jus J1 a eu une concentration en sucres basse, de 38,77 g/kg. Pour l'augmenter, un recyclage de J1 (20 kg/h) vers le bac de J2 a été installé et le mélange des jus J1 et J2 a été chauffé et recyclé vers le mélangeur M. Pour améliorer le vide et fermer le gâteau qui tendait à se fissurer, la première buse de lavage était par aspersion (zone 4) et la deuxième par déversement (zone 6). En effet, 70 kg/h de jus de J1 à 53-57 g/kg de sucres a été produit. Les conditions opératoires sont détaillées dans le tableau 4 suivant.

### Exemple 4 selon l'invention : Biomasse à base de paille

Cet essai reprend la même configuration que l'exemple 3, également avec une biomasse à base de paille donc, aux différences suivantes : ici la presse finale (pressant le gâteau de marc lavé au niveau de la zone 9 du filtre à bande) n'est pas utilisée, la première buse de lavage est utilisée par déversement et par non aspersion, et la deuxième buse de lavage est utilisée par aspersion et non déversement.

Le jus J1 produit possède une concentration en sucres plus élevée (50-66 g/kg de sucre). Les conditions opératoires sont détaillées dans le tableau 4 suivant.

### Exemple 5 selon l'invention : Biomasse à base de TCR

Cet essai reprend la même configuration que l'exemple 1, également avec une biomasse à base de TCR donc, avec les différences suivantes : ici, la presse finale (pressant le gâteau de marc lavé au niveau de la zone 9 du filtre à bande) n'est pas utilisée, et la première buse de lavage est utilisée par déversement et non aspersion.

Le jus J1 produit possède une concentration de 51 g/kg de sucre. Les conditions opératoires sont détaillées dans le tableau 4 suivant.

Pour vérifier rapidement sur site si la concentration en sucres dans le jus J1 atteint bien les 50g/kg comme demandé, des mesures ont été effectuées avec un réfractomètre PAL-1 de marque ATAGO.

Le tableau 4 ci-dessous présente les caractéristiques de charge: un mélange de M0 avec les jus J2 (ou J1+J2 pour la paille) et les produits J1 et M0 et quantifie l'efficacité de la séparation obtenue avec l'invention : la séparation de la paille a été le plus performant (99%)

**Tableau 4**

| | | **Ex 5 : TCR (2)** | **Ex 4 : Paille (2)** | **Ex 3 : Paille** | **Ex 2 : Miscanthus** | **Ex 1 : TCR** |
|---|---|---|---|---|---|---|
| **Charge** | Biomasse prétraitée M0 | 44% solide | 29-32% solide | 29 - 30% solide | 42% solide | 38 - 43% solide |
| | | 53%MS | 41-46% MS | 42 - 44% MS | 54% MS | 49 - 53% MS |
| | | 80 g/kg de sucre | 65-93 g/kg de sucre | ∼ 90 g/kg de sucre | ∼ 95 g/kg de sucre | ∼ 83 g/kg de sucre |
| | Jus recycle J2 | ∼ 0% solide | - 0% solide | - 0.003% solide | - 0% solide | - 0% solide |
| | | 4%MS | 5-11 %MS | 6% MS | 4%MS | 8.6 % MS |
| | | 23 g/kg de sucre | 22-37 g/kg de sucre | - 33 g/kg de sucre | - 27 g/kg de sucre | ∼ 50 g/kg de sucre |
| | Biomasse prétraitée M0Re | 12% solide | 8.3% solide | 8.5% solide | 18% solide | 16 % solide |
| | | 18% MS | 18% MS | 16 % MS | 26 % MS | 25 % MS |
| **Produit** | J1 | ∼ 0.04 % solide | ∼ 0.04 % solide | - 0.03 % solide | - 0.09 % solide | ∼ 0.07 % solide |
| | | 8%MS | 10-11% MS | 9 - 10% MS | 9 - 10% MS | 11% MS |
| | | 51 g/kg de sucre | 50-66 g/kg de sucre | 53 - 57 g/kg de sucre | 58 - 60 g/kg de sucre | 69 - 70 g/kg de sucre |
| | M1 | 38% solide | 23-26% solide | 30 - 32% solide | 31 - 33% solide | 33 - 34 % solide |
| | | 40%MS | 28-30% MS | 32 - 33 % MS | 37 - 38 % MS | 35 - 37 % MS |
| | | 26 g/kg de sucre | 19 - 36 g/kg de sucre | 0.3 - 1 g/kg de sucre | 32-38 g/kg de sucre | 13-20 g/kg de sucre |
| **Efficacité de la séparation** | E | ∼ 85% | ∼ 71-77% | 99% | ∼ 62% | ∼ 79% |

En conclusion, on voit que ces cinq exemples selon l'invention, avec des biomasses initiales différentes, parviennent à atteindre l'objectif d'un jus extrait J1 avec la concentration en sucres voulue, largement au-dessus du seuil minimal fixé de 50 g/kg. La séparation selon l'invention avec les deux outillages en série, mélangeur en continu et filtre (à bande) en continu permet beaucoup de flexibilité, et une grande compacité. Ils peuvent donc s'insérer sans difficulté dans une installation de traitement de biomasse à différentes étapes, dès qu'on a besoin d'opérer une séparation solide/liquide sur un mélange en souhaitant valoriser aussi bien la partie solide que la partie liquide obtenues après séparation.

L'invention permet ainsi, quelle que soit la biomasse prétraitée et ses contraintes rhéologiques, d'obtenir un jus sucré d'une concentration supérieure à 50 g/kg, ce qui n'est pas le cas lorsque l'invention n'est pas mise en oeuvre, comme montré dans les exemples comparatifs suivants :

### Exemples comparatifs (non conforme à l'invention) :

Les biomasses prétraitées de deux des exemples précédents sont mises en oeuvre dans une configuration non conforme à l'invention : le substrat M0 est mélangé dans un mélangeur M avec de l'eau puis séparée sur le filtre à bande F. Dans cette mise en oeuvre non conforme à l'invention, il n'y a pas de recyclage d'un flux extrait du filtre à bande F vers le mélangeur M.

### Exemple 6 comparatif : Biomasse à base de TCR

La biomasse prétraitée M0 TCR issue de l'exemple 1 est mélangé dans un mélangeur M avec de l'eau puis séparée sur le filtre à bande F lui-même opéré selon la configuration de l'exemple 1. De par sa rhéologie, la biomasse prétraitée TCR doit être diluée à un taux de solides inférieur à 16% pour former un gâteau apte à être filtré sur le filtre à bande F. On rappelle la composition du marc M0 de TCR de l'exemple 1 : teneur en solide 38%, teneur en sucres 83 g/kg. Pour obtenir une rhéologie correcte en sortie de mélangeur, il est nécessaire d'abaisser la teneur en solide du substrat après mélange, il est ainsi nécessaire d'ajouter 68,8 kg d'eau à 50 kg de marc M0 dans le mélangeur, ce mélange est ensuite déposé sur le filtre à bande. La première zone du filtre à bande permet de sortir un jus concentré J1 a une teneur de 41,6 g / kg de sucres seulement. Dans cet exemple non-conforme à l'invention, on voit qu'il n'est pas possible d'atteindre une concentration de 50 g/ kg de sucres dans le jus J1 pour le substrat TCR.

### Exemple 7 comparatif : Biomasse à base de paille

La biomasse prétraitée M0 paille issue de l'exemple 3 est mélangé dans un mélangeur M avec de l'eau puis séparée sur le filtre à bande F lui-même opéré selon la configuration de l'exemple 3. De par sa rhéologie, la biomasse prétraitée Paille doit être diluée à un taux de solides inférieur à 9% pour former un gâteau apte à être filtré sur le filtre à bande F. On rappelle la composition du marc M0 de paille de l'exemple 3 : teneur en solide 30%, teneur en sucres 90 g/kg. Pour obtenir une rhéologie correcte en sortie de mélangeur, il est nécessaire d'abaisser la teneur en solide du substrat après mélange, il est ainsi nécessaire d'ajouter 117 kg d'eau à 50 kg de marc M0 dans le mélangeur, ce mélange est ensuite déposé sur le filtre à bande. La première zone du filtre à bande permet de sortir un jus concentré J1 a une teneur de 27 g / kg de sucres. Dans cet exemple non-conforme à l'invention, on voit qu'il n'est pas possible d'atteindre une concentration de 50 g/ kg de sucres dans le jus J1 pour le substrat paille.

## Revendications

1. Procédé de traitement d'une biomasse lignocellulosique, ledit procédé comprenant au moins l'étape suivante :
**a.** Prétraitement de la biomasse par au moins une opération de cuisson ou d'explosion à la vapeur, afin d'obtenir un substrat prétraité,
**caractérisé en ce qu'**il comprend également au moins une étape de :
**b.** Séparation liquide/solide sur au moins une partie du substrat prétraité obtenu à l'issue de l'étape **a** ou à l'issue d'une étape additionnelle de traitement dudit substrat après l'étape **a,** ladite étape de séparation comprenant deux sous-étapes successives :
- une sous-étape amont de contactage **b1** du mélange solide/liquide mise en oeuvre par un mélangeur (M) en continu utilisant un fluide de mélange, éventuellement précédé d'une pompe, et
- une sous-étape aval **b2** d'extraction/lavage mise en oeuvre par un filtre en continu, notamment un filtre à bandes (F), utilisant un fluide de lavage, pour obtenir une phase solide enrichie en solide et une pluralité de phases liquides enrichies en liquide, avec un recyclage au moins partiel d'une phase liquide extraite du filtre en entrée du mélangeur en tant que fluide de mélange.

2. Procédé selon la revendication précédente, **caractérisé en ce que** le filtre en continu fonctionne à contre-courant entre la circulation du mélange solide/liquide à séparer et le fluide d'extraction/lavage.

3. Procédé selon l'une des revendications précédentes, **caractérisé en ce qu'**on réalise la séparation du mélange solide/liquide lors de l'étape **b** sur une première partie du substrat prétraité obtenu à l'issue de l'étape **a, en ce qu'**on soumet une seconde partie dudit substrat prétraité à au moins une étape ultérieure de traitement, notamment une hydrolyse, et **en ce qu'**on soumet également la phase solide enrichie en solide obtenue à l'étape **b** à au moins une étape ultérieure de traitement, notamment la même ou les mêmes que celles subies par la seconde partie du substrat prétraité.

4. Procédé selon l'une des revendications précédentes, **caractérisé en ce qu'**il comprend une étape **e** d'hydrolyse enzymatique du substrat prétraité issu de l'étape **a** de prétraitement pour obtenir un hydrolysat, **en ce qu'**on réalise la séparation solide/liquide **b** sur au moins une partie dudit hydrolysat.

5. Procédé selon l'une des revendications précédentes, **caractérisé en ce qu'**on réalise la séparation solide/liquide **b** sur au moins une partie, notamment tout, du substrat prétraité obtenu à l'issue de l'étape **a** de prétraitement pour obtenir une phase solide enrichie en solide, et **en ce qu'**il comprend une étape **e** d'hydrolyse enzymatique de ladite phase solide enrichie en solide pour obtenir un hydrolysat.

6. Procédé selon l'une des revendications précédentes, **caractérisé en ce que** les phases liquides enrichies en liquide issues de l'étape de séparation **b** sont des jus sucrés, **en ce que** ledit procédé comprend aussi une étape **c** de production d'enzymes et/ou une étape **d** de production de levures, et **en ce que** au moins un desdits jus sucré est utilisé pour ladite production d'enzymes **c** ou de levures **d.**

7. Procédé selon l'une des revendications précédentes, **caractérisé en ce qu'**il comprend également les étapes suivantes après l'étape **a** de prétraitement :
**e.** Hydrolyse enzymatique du substrat prétraité, afin d'obtenir un hydrolysat comportant une matière solide et une phase liquide contenant des sucres,
**f.** Fermentation de l'hydrolysat, afin d'obtenir un vin de fermentation,
l'étape **f** de fermentation pouvant succéder ou être concomitante à l'étape **e** d'hydrolyse enzymatique
**g.** Séparation/Distillation du vin de fermentation, afin d'obtenir un produit de distillation sous forme d'un alcool, d'un solvant, ou autre molécule biosourcée, et des vinasses,
l'étape **b** étant réalisée sur au moins une partie des produits obtenus après l'étape **e** d'hydrolyse et/ou **f** de fermentation et/ou **g** de séparation/distillation.

8. Procédé selon l'une des revendications précédentes, **caractérisé en ce que**, dans l'étape **b** de séparation, on chauffe le fluide d'extraction/lavage avant introduction dans le filtre (F) et/ou la phase liquide extraite du filtre et recyclée avant son introduction dans le mélangeur, notamment à une température d'au moins 30 °C, notamment d'au plus 90°C, notamment une température comprise entre 40°C et 80°C.

9. Procédé selon l'une des revendications précédentes, **caractérisé en ce que**, dans l'étape **b**, le filtre est un filtre à bande (F) qui comprend au moins deux, notamment au moins trois, zones successives, avec soutirage dans chaque zone d'une phase liquide.

10. Procédé selon la revendication précédente, **caractérisé en ce que** la phase liquide de la première et/ou de la deuxième zone est recyclée au moins en partie en entrée du mélangeur (M) de l'étape **b**, et **en ce que** la phase liquide soutirée de la troisième et/ou de la dernière zone est recyclée au moins en partie en tant que fluide de lavage/extraction du filtre à bande (F).

11. Procédé selon l'une des revendications précédentes, **caractérisé en ce qu'**on sélectionne les paramètres de fonctionnement des étapes **b1** et **b2** de séparation en fonction des caractéristiques du mélange solide/liquide pour que les phases liquides produites extraites du filtre à bande (F) aient une concentration d'au moins 50 g de produit /kg, notamment au moins 50 g de sucre/kg quand la séparation **b** est réalisée sur le substrat prétraité issu de l'étape **a** de prétraitement.

12. Installation de traitement d'une biomasse lignocellulosique, **caractérisée en ce qu'**elle comprend au moins :
- une zone de prétraitement de la biomasse par au moins une opération de cuisson ou d'explosion à la vapeur, afin d'obtenir un substrat prétraité,
- une zone de séparation liquide/solide sur au moins une partie du substrat prétraité obtenu en sortie de la zone de prétraitement ou d'une zone additionnelle de traitement dudit substrat après la zone de prétraitement, ladite zone de séparation comprenant deux sous-zones en série: - une sous-zone amont de contactage comprenant un mélangeur (M) en continu utilisant un fluide de mélange, éventuellement associé à une pompe amont, et - une sous-zone aval d'extraction/lavage comprenant un filtre en continu, notamment un filtre à bandes (F), avec un fluide de lavage, avec un fonctionnement du filtre de préférence en contre-courant entre la circulation du mélange solide/liquide à séparer et le fluide d'extraction/lavage, et un recyclage au moins partiel d'une phase liquide extraite par le filtre en entrée du mélangeur en tant que fluide de mélange.

13. Installation de traitement selon la revendication précédente, **caractérisée en ce qu'**elle prévoit, dans la zone de séparation : - des moyens de connexion fluidique entre le filtre (F) et le mélangeur (M) pour recycler au moins une partie d'une phase liquide extraite par le filtre en entrée du mélangeur et - des moyens de connexion fluidique pour recycler une autre phase liquide extraite du filtre (F) en tant que fluide de lavage dudit filtre.

14. Installation de traitement selon l'une des revendications 12 ou 13, **caractérisée en ce qu'**elle comprend également une zone de production d'enzymes et/ou une zone de production de levures, et **en ce qu'**elle prévoit un moyen de transfert d'au moins une phase liquide extraite du filtre à bande depuis la zone de séparation à la zone de production d'enzymes et/ou à la zone de production de levures, ladite phase liquide étant un jus sucré.

15. Utilisation du procédé ou de l'installation selon l'une des revendications précédentes pour le traitement de biomasses du type bois, paille, résidus agricoles, et toutes cultures énergétiques dédiées, notamment des plantes annuelles ou pluriannuelles telles que le miscanthus en vue de produire des sucres, des biocarburants ou des molécules biosourcées.
